Europäisches Patentamt

(19) European Patent Office

Office européen des brevets

(11) Numéro de publication: **0 078 734**
**B1**

(12) **FASCICULE DE BREVET EUROPÉEN**

(45) Date de publication du fascicule du brevet:
15.10.86

(51) Int. Cl.⁴: **G 01 N 33/541,** G 01 N 33/74, G 01 N 33/545

(21) Numéro de dépôt: 82401955.8

(22) Date de dépôt: 25.10.82

(54) **Procédé de dosage immunologique mettant en oeuvre deux anticorps, le deuxième anticorps étant purifié par chromatographie d'affinité, supports pour sa mise en oeuvre.**

(30) Priorité: 29.10.81 FR 8120347

(43) Date de publication de la demande:
11.05.83 Bulletin 83/19

(45) Mention de la délivrance du brevet:
15.10.86 Bulletin 86/42

(84) Etats contractants désignés:
AT BE CH DE GB IT LI LU NL SE

(56) Documents cité:
EP-A-0 058 780
WO-A-81/02792
FR-A-2 322 373
FR-A-2 385 099
US-A-4 021 534
US-A-4 048 298
US-A-4 343 896

CLINICAL CHEMISTRY, vol. 27, no. 6, juin 1981, pages 896-900, Pennsylvania (USA); P.CORNALE et al.: "Column affinity chromatography for bound/free separation in ligand assays. I. Radioimmunoassay of choriomammotropin (human placental lactogen)"
STEROIDS, vol. 38, no. 4, octobre 1981, pages 453-463, San Francisco (USA); H.ARAKAWA et al.: "Chemiluminescence enzyme immunoassay of dehydroepiandrosterone and its sulfate using

(73) Titulaire: BIOMERIEUX, Société Anonyme dite, Marcy l'Etoile, F-69260 Charbonnières- les- Bains (FR)

(72) Inventeur: Trouyez, Gérard, 30, rue Chaziere, F-69004 Lyon (FR)

(74) Mandataire: Gillard, Marie- Louise, Cabinet Beau de Loménie 55, Rue d'Amsterdam, F-75008 Paris (FR)

(56) Documents cité: (suite)
peroxidase as label".
Sokolowski-wood: Radioimmunoassay in Theorie und Praxis page 95

## Description

La présente invention concerne un procédé de dosage immunologique mettant en oeuvre deux anticorps, le deuxième anticorps étant purifié par chromatographie d'affinité. Elle concerne aussi les supports pour sa mise en oeuvre qui ont le plus souvent la forme d'un tube en matière plastique, lesdits supports étant revêtus du deuxième anticorps hautement purifié par chromatographie d'affinité; l'invention a également pour objet un procédé de préparation de ces supports et en particulier de tubes. Ceux-ci, qui peuvent être fournis numérotés et disposés sur des portoirs de manière à être prêts à l'emploi, sont stables au moins 15 jours à 37°C et 6 mois à +4°C dans leur emballage.

Ils permettent de simplifier la réalisation des dosages immunologiques d'antigènes et d'haptènes.

On a déjà proposé d'utiliser des anticorps purifiés immobilisés sur un support. Par example, la demande de brevet WO81/02792 concerne un procédé de détection par compétition classique, selon lequel on met en contact une matrice solide, sur laquelle on a immobilisé un anticorps purifié, avec le lait à tester et un antibiotique marqué avec une enzyme.

Dans de nombreuses méthodes de dosage immunologique et plus particulièrement radio-immunologique, on utilise un deuxième anticorps dirigé contre le premier anticorps et donc réagissant avec lui, pour séparer l'antigène libre de celui lié au premier anticorps.

Dans les méthodes immunoprécipitantes, le deuxième anticorps est utilisé à l'état libre (Morgan et Lazarow 1962, Utiger et Col. 1962).

Dans les méthodes d'immunoprécipitation accélérée, un accélérateur de précipitation (poly-éthylèneglycol, éthanol, sulfate d'ammonium etc) est ajouté au deuxième anticorps, de manière à réduire la durée de l'immunoprécipitation à 15 ou 30 minutes (Martin et Landon 1974).

Dans les méthodes utilisant un immuno-adsorbant microparticulaire, le deuxième anticorps est soit:

— préincubé à un premier anticorps;
— couplé à de la cellulose microcristalline ou à du Sépharose® 4B (DASP) selon WIDE, à du gel d'acrylamide, à des particules de latex etc;
— couplé à des polymères associés à de l'oxyde de fer (Nye) et col. 1976).

A ce type de méthode doivent être assimilées les techniques faisant appel à la protéine A du staphylocoque doré qui joue un rôle comparable au deuxième anticorps (Jonsson et Kronvall 1973).

Toutes ces méthodes présentent deux inconvénients essentiels:

— le piperage d'un réactif en suspension constitue souvent une source d'hétérogénéité dans les dosages. Seules les méthodes immunoprécipitantes simples ne présentent pas ce premier inconvénient;
— la nécessité de faire appel à une étape de centrifugation ou de microfiltration pour séparer la fraction d'antigène libre de celle liée. Seule la méthode utilisant des particules d'oxyde de fer évite cet inconvénient en réalisant la séparation dans un champ magnétique; cela impique par contre la nécessité de se procurer des équipements supplémentaires.

La transposition des méthodes en phase solide selon Catt et Tregear (1967) au couplage du deuxième anticorps supprime ce deuxième inconvénient.

Dans la méthode RIA, le deuxième anticorps (Ab$^2$) est fixé sur la phase solide, et celle-ci est incubée avec une solution du premier anticorps (Ab) et la solution contenant l'antigène à doser (Ag) à laquelle on ajoute ensuite l'antigène marqué (Ag*). Il se forme des complexes Ab-Ag et Ab-Ag* qui se fixent au Ab$^2$ sur la phase solide, l'antigène (marqué et non-marqué) non-combiné restant dans la phase liquide.

Le deuxième anticorps est habituellement obtenu par injections répétées du plasma ou du sérum sanguin d'un animal ou de l'homme à un animal d'une autre espèce: le sérum de celui-ci contient un anticorps (gamma-globulines) dirigé contre les gamma-globulines du premier animal ou de l'homme. Les gamma-globulines (Ab$^2$) du sérum de l'animal immunisé doivent être séparées et purifiées avant d'être fixées sur la phase solide.

Dans la méthode de ZIOLA B. R. et col. (1977), les gamma-globulines du deuxième anticorps (sérum de mouton, antigamma-globuline de lapin) sont purifiées seulement par une précipitation au sulfate de sodium suivie d'une filtration moléculaire sur gel Sephadex® G-200 avant d'être adsorbées sur des billes de polystyrène. Cependant, la manipulation des billes manque de praticabilité par rapport à celle des tubes. De plus, elles représentent un réactif supplémentaire à manipuler.

Dans la méthode de Cocola F. et col. (1973), les gamma-globulines du deuxième anticorps (sérum de chèvre, anti-gamma-globuline de lapin) sont purifiées seulement par une précipitation au sulfate d'ammonium suivie d'une chromatographie sur DEAE cellulose avant d'être adsorbées sur des tubes en polystyrène. Ces tubes ont servi uniquement pour le dosage de l'hormone chorionique somatotrope (HCS) selon un mode opératoire caractérisé par des incubations à 37°C et une addition séquentielle des réactifs (pipetage successif du sérum anti-HCS et des échantillons à doser, suivie de 4 heures d'incubation à 37°C puis d'une addition de HCS marqué à l'oide 125, suivie d'une incubation de 14 heures à 37°C). La stabilité de ces tubes ne dépasse pas 2 mois à +4°C. De plus, ce travail n'a pas permis le développement de réactifs commerciaux.

Dans le procédé selon l'invention, les gamma-globulines du deuxième anticorps sont purifiées par chromatographie d'affinité avant d'être fixées sur les supports, en particulier les tubes qui sont de préférence en polyéthylène. Il en résulte l'obtention d'immunoadsorbants beaucoup plus performants se prêtant lors des dosages:

— à l'addition en une seule étape, sans incubation intermédiaire, de tous les réactifs successivement, le premier anticorps étant ajouté en dernier en tant que réactif déclenchant;
— à une incubation durant de quelques heures (méthode en cinétique) à 24 heures (méthode à l'équilibre) à la température du laboratoire, ou autre température.

L'invention a aussi pour objet les supports en matière plastique revêtus d'un deuxième anticorps pour la mise en oeuvre du procédé selon l'invention. Elle concerne également la préparation desdits supports revêtus d'un deuxième anticorps. Ce procédé comprend deux étapes:

1) Obtention des gamma-globulines du deuxième anticorps hautement purifiées par chromatographie d'affinité sur une colonne constituée par un gel sur lequel ont été fixées les gamma-globulines contre lesquelles est dirigé l'anticorps à purifier;
2) fixation de ces gamma-globulines sur les tubes en polymères (polyéthylène, polystyrène et autres).

Première étape: Exemple de purification de gamma-globulines de sérum de mouton anti-gamma-globulines de lapin:

On utilise, pour la chromatographie d'affinité, un gel (de préférence un gel de Sepharose® 4B ou d'Ultragel® ACA 22) sur lequel ont été fixés des gamma-globulines de lapin.

La chromatographie d'affinité proprement dite s'effectue alors de la façon suivante: On introduit le sérum de mouton (100 ml par exemple) anti-gamma-globulines de lapin dans la colonne et on élue par un tampon phosphate de sodium à pH 7,4 jusqu'au retour de la ligne de base du chromatogramme. Les gamma-globulines de mouton anti-gammaglobulines de lapin sont ensuite éluées par un tampon apportant une variation brutale de pH et de conductivité de la colonne (par exemple, un tampon glycine-soude de pH 2,8).

Après équilibrage de l'éluat à pH 7,4, on le fixe sur les tubes.

Deuxième étape: Exemple de fixation par adsorption sur des tubes en polyéthylène de gamma-globulines de mouton anti-gammablobulines de lapin purifiées par chromatographie d'affinite:

On fait incuber l'éluat obtenu précédemment (30 à 100 µl/ml) dans chacun des tubes; ceux-ci sont ensuite rincés successivement par une solution de chlorure de sodium à 9‰ et par une

solution protéique. Les tubes ainsi traités sont séchés par lyophilisation ou tout autre procédé avant d'être conditionnés en sachet en présence d'un déshydratant.

L'originalité de ces tubes tient:

— à leur stabilité dans leur conditionnement au moins 15 jours à 37°C et 6 mois à +4°C;
— à leur performance de reproductibilité dans les dosages.

Les supports revêtus d'un deuxième anticorps, selon l'invention, sont utilisés pour les dosages immunologiques et plus particulièrement radio-immunologiques, d'hormones telles que l'hormone folliculostimulante humaine (hFSH) et l'hormone lutéinisante humaine (hLH).

Premier exemple d'application des tubes selon l'invention au dosage ratio-immunologique de la hFSH:

Le système de réactifs contient:

2 flacons de réactif R1: $^{125}$I-hFSH ($\leqslant$2,78 10$^4$ Bq=0,75 µ Ci)
7 flacons de réactif R2: hFSH étalon à des concentrations comprises entre 0 et 40 ng/ml
2 flacons de réactif R3: sérum de lapin anti-hFSH
2 portoirs de 48 tubes selon l'invention de réactif R4: (gamma-globulines de mouton anti-gamma-globulines de lapin)
1 flacon de réactif R5: diluant (sans hFSH)
1 flacon de réactif R6: tampon PBS-BSA pH 7,4→50 ml
1 flacon de réactif R7 } sérums de contrôle
1 flacon de réactif R8 } humain

Deuxième exemple d'application des tubes selon l'invention au dosage ratio-immunologique de la hLH:

Le système de réactifs contient:

2 flacons de réactif R1: $^{125}$I-hLH ($\leqslant$2,78 10$^4$Bq=0,75 µ Ci)
7 flacons de réactif R2: hLH étalon à des concentrations comprises entre 0 et 40 ng/ml
2 flacons de réactif R3: sérum de lapin anti-hLH
2 portoirs de 48 tubes selon l'invention de réactif R4: (gamma-globulines de mouton anti-gamma-globulines de lapin)
1 flacon de réactif R5: diluant (sans hLH)
1 flacon de réactif R6: tampon PBS-BSA pH 7,4→50 ml
1 flacon de réactif R7 } sérums de contrôle
1 flacon de réactif R8 } humain

L'originalité de ces deux systèmes de réactifs tient:

— à la simplicité de leur mode opératoire:
. tous les réactifs sont ajoutés successivement en une seule étape, sans incubation intermédiaire, le premier anticorps (anti-hLH ou anti-hFSH) étant ajouté en dernier;

.l'incubation est de quelques heures à 24 heures à la température du laboratoire. Il n'y a pas d'étape de centrifugation.
— à la conception du dosage:

. le fait d'ajouter le premier anticorps en dernier, contrairement au protocole de Cocola permet à ce réactif de jouer un rôle de réactif déclenchant: ainsi, les réactions immunologiques du dosage démarrent au même moment dans tous les tubes. Le dosage peut ainsi être fait en cinétique ou à l'équilibre. Cet avantage existe de la même manière vis-à-vis des méthodes en tubes revêtus pour lesquelles un premier anticorps et non un deuxième anticorps est fixé au fond des tubes;

. l'absence d'absorption des traceurs $^{125}$I-hLH ou $^{125}$I-hFSH dans les tubes coat RIA dans les conditions du dosage ainsi que l'aspiration du milieu réactionnel en fin d'incubation suivie d'un rinçage permet d'obtenir des "non-spécifiques" inférieurs à 1%.

L'emploi des supports selon l'invention peut être étendu au dosage radio-immunologique, enzymo-immunologique, colorimétrique ou fluorescent de produits tels que:

— les haptènes: stéroïdes, vitamines, médicaments.
— les macromolécules: protéines telles que des hormones (prolactine, hormone thyréostimulante...) des protéines sériques (protéines liantes, anticorps...) des protéines tissulaires dans les matériaux biologiques tels que le sérum, le plasma, l'urine, des virus végétaux.

Pour faciliter l'emploi des tubes selon l'invention, ils peuvent être regroupés dans les systèmes de réactifs ou "kits" (pour les dosages immunologiques), sur des portoirs réalisés en matériaux plastiques, de préférence thermoformés.

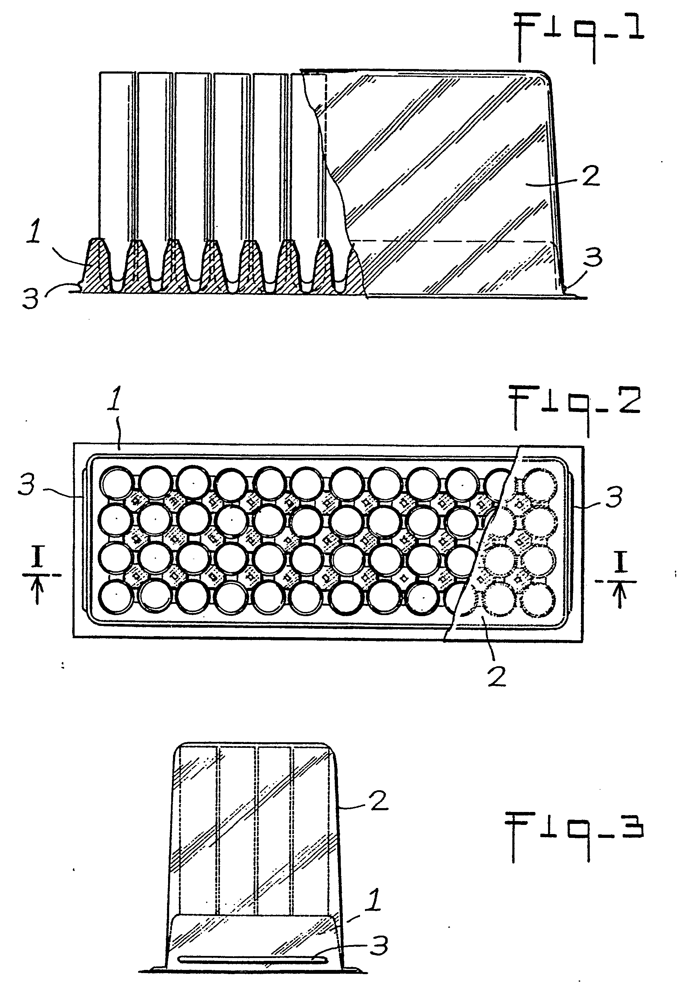

Une forme de réalisation d'un portoir de tubes selon l'invention est représentée dans les figures 1, 2 et 3 ci-après.

La figure 2 représente une vue de dessus, la figure 3 une vue transversale et la figure 1 une coupe longitudinale selon l'axe I—I.

Le portoir est constitué d'une base 1 présentant des alvéoles contiguës, circulaires, ayant un fin canal de haut en bas destiné à faciliter l'introduction des tubes, et d'un couvercle 2 également réalisé en matériaux plastiques, de préférence thermoformés, venant s'enclipser dans la base, grâce aux joncs d'enclipsage 3. On constitue ainsi un ensemble compact ayant les avantages suivants:

a) les tubes étant jointifs sont parfaitement alignés dans le portoir, facilitant ainsi les opérations de pipetage et de lavage.

b) La bonne tenue de ces tubes premier leur manipulation aisée et notamment retournement pour toute opération de vidange et égouttage.

c) Le couvercle disposable peut être maintenu en position haute pour permette le séchage par lyophilisation ou tout autre moyen. Le clipsage peut alors se faire dans la machine même par rapprochement des plateaux supports. De plus, un gaz inerte peut être introduit dans l'emballage afin de protéger le produit jusqu'à sa mise en conditionnement étanche définitif.

d) La forme de coque du couvercle offre la possibilité d'operculer au moyen d'un film barrière thermosoudable. Un blister étanche est ainsi réalisé.

e) Les tubes, au nombre de 48, sont numérotés et prêts à l'emploi. Ce sont des tubes à hémolyse en polyéthylène, polystyrène ou toute autre matière plastique.

f) Le modèle de portoir est applicable à toute forme de tube, ou contenant, destiné à das répartitions et lavages en série.

En résumé, ou obtient ainsi selon l'invention des tubes prêts à l'emploi, c'est-à-dire secs, numérotés et disposés en portoirs, ce qui amélroire de manière considérable la mise en oeuvre des dosages immunologiques.

**Revendications**

1. Procédé de dosage immunologique d'antigènes mettant en oeuvre deux anticorps, consistant à fixer un deuxième anticorps sur une phase solide, à faire incuber celle-ci avec une solution du premier anticorps et la solution contenant l'antigène à doser, à laquelle on ajoute ensuite l'antigène marqué, caractérisé en ce que l'on utilise un deuxième anticorps insolubilisé qui a été préalablement purifié par chromatographie d'affinité.

2. Procédé selon la revendication 1, caractérisé en ce que les réactifs sont ajoutés en une seule étape sans incubation intermédiaire, le premier anticorps étant ajouté en dernier et servant de réactif déclenchant.

3. Procédé selon l'une des revendication 1 ou 2, caractérisé en ce que l'on dose l'hormone folliculo-stimulante et l'hormone lutéinisante humaines.

4. Procédé selon l'une quelconque des revendications 1 à 3, caractérisé en ce que l'on réalise le dosage aussi bien à l'éuilibre qu'en cinétique.

5. Supports en matière plastique revêtus d'un deuxième anticorps, pour la mise en oeuvre du procédé selon la revendication 1, caractérisés en ce que le revêtement est constitué par un deuxième anticorps hautement purifié par chromatographie d'affinité, fixé par adsorption sur un polymère, de préférence on polyéthylène.

6. Supports selon la revendication 5, caractérisé en ce que le deuxième anticorps est une gamma-globuline du sérum d'une espèce animale, anti-gamma-globuline du sérum d'une autre espèce animale ou de l'homme, purifiée par chromatographie sur des gamma-globulines de la deuxième espèce animale ou de l'homme.

7. Supports selon l'une des revendications 5 et 6, caractérisés en ce que le deuxième anticorps est une gamma-globuline de mouton anti-gamma-globuline de lapin.

8. Supports selon l'une des revendications 5 à 7, caractérisés en ce qu'ils ont la forme de tubes.

9. Procédé de préparation du supports selon l'une des revendications 5 à 8, caractérisé en ce qu'on traite le sérum immun contenant le deuxième anticorps par chromatographie d'affinité sur des gamma-globulines contre lesquelles est dirigé l'anticorps à purifier et que le deuxième anticorps purifié ainsi obtenu est fixé par adsorption sur le support en une matière plastique telle que polyéthylène ou polystyrène.

10. Procédé selon la revendication 9, caractérisé en ce que, pour la chromatographie d'affinité, on utilise une colonne remplie d'un gel sur lequel sont fixées des gamma-globulines contre lesquelles est dirigé l'anticorps à purifier, et on fait passer sur cette colonne le sérum immun contenant l'anticorps à purifier, puis on élue par un tampon phosphate à pH 7,4 jusqu'au retour de la ligne de base du chromatogramme; on élue ensuite les gamma-globulines du deuxième anticorps par un tampon apportant une variation brutale de pH et de conductivité de la colonne.

11. Procédé selon l'une des revendications 9 et 10 caractérisé en ce que l'éluat contenant les gamma-globulines du deuxième anticorps est équilibré à pH 7,4, dilué et incubé sur le support et en particulier dans les tubes où il se fixe par adsorption sur le polymère constituant la paroi du tube.

12. Dispositif de présentation ou portoir pour les tubes selon la revendication 8, caractérisé en ce qu'il est constitué par une matière plastique thermoformée et comprend une base (1) présentant des alvéoles contiguës, circulaires, ayant un fin canal de haut en bas pour l'introduction des tubes et un couvercle (2) venant s'enclipser dans la base grâce à deux joncs d'enclipsage (3) qui permettent au couvercle d'être maintenu en position haute pour le séchage des tubes ou l'introduction d'un gaz de protection ou en position basse pour operculer les tubes et permettre leur retournement.

**Patentansprüche**

1. Verfahren zur immunologischen Bestimmung von Antigenen unter Anwendung zweier Antikörper, bei welchem ein zweiter Antikörper auf einer Festphase fixiert wird, diese mit einer Lösung des ersten Antikörpers und der das zu bestimmende Antigen enthaltenden Lösung inkubiert wird, zu welcher man danach das markierte Antigen zugibt, dadurch gekennzeichnet, daß man einen zweiten, unlöslich gemachten Antikörper verwendet, welcher zuvor mittels Affinitätschromatographie gereinigt worden ist.

2. Verfahren nach Anspruch 1, dadurch gekennzeichnet, daß die Reagenzien ohne Zwischeninkubation in einem einzigen Schritt zugegeben werden, wobei der erste Antikörper als letzter zugegeben wird und als auslösendes Reagens fungiert.

3. Verfahren nach einem der Ansprüche 1 oder 2, dadurch gekennzeichnet, daß man das follikelanregende Hormon und das Luteinisierende Hormon des Menschen bestimmt.

4. Verfahren nach einem der Ansprüche 1 bis 3, dadurch gekennzeichnet, daß man die Bestimmung sowohl im Gleichgewichtszustand als auch kinetich durchführt.

5. Mit einem zweiten Antikörper überzogene Träger aus Plastikmaterial zur Durchführung des Verfahrens nach Anspruch 1, dadurch gekennzeichnet, daß der Überzug aus einem mittels Affinitätschromatographie hochgereinigten zweiten Antikörper besteht, der durch Adsorption auf einem Polymerisat, vorzugsweise Polyäthylen, fixiert ist.

6. Träger nach Anspruch 5, dadurch gekennzeichnet, daß der zweite Antikörper ein Gammaglobulin des Serums einer Tiergattung, Anti-Gammaglobulin des Serums einer anderen Tiergattung oder des Menschen, gereinigt mittels Chromatographie über Gammaglobulinen der zweiten Tiergattung oder des Menschen, ist.

7. Träger nach einem der Ansprüche 5 und 6, dadurch gekennzeichnet, daß der zweite Antikörper ein Schaf-Gammaglobulin-Kaninchen-Antigammaglobulin ist.

8. Träger nach einem der Ansprüche 5 bis 7, dadurch gekennzeichnet, daß sie Röhrenform haben.

9. Verfahren zur Herstellung von Trägern nach einem der Ansprüche 5 bis 8, dadurch gekennzeichnet, daß man das den zweiten Antikörper enthaltende Immunserum mittels Affinitätschromatographie über Gammaglobulinen behandelt, gegen die der zu reinigende Antikörper gerichtet ist, und daß der so erhaltene, gereinigte zweite Antikörper auf dem Träger aus Plastikmaterial, wie Polyäthylen oder Polystyrol, mittels Adsorption fixiert wird.

10. Verfahren nach Anspruch 9, dadurch gekennzeichnet, daß man für die Affinitätschromatographie eine mit einem Gel gefüllte Säule verwendet, auf welchem Gammaglobuline fixiert sind, gegen die der zu reinigende Antikörper gerichtet ist, und daß man das den zu reinigenden Antikörper enthaltende Immunserum über die Säule laufen läßt, danach mittels eines Phosphatpuffers mit pH 7,4 bis zur Rückkehr der Basislinie des Chromatogramms eluiert und daraufhin die Gammaglobuline des zweiten Antikörpers mittels eines Puffers eluiert, der eine kräftige Veränderung des pH-Werts und der Leitfähigkeit der Säule verursacht.

11. Verfahren nach einem der Ansprüche 9 und 10, dadurch gekennzeichnet, daß das die Gammaglobuline des zweiten Antikörpers enthaltende Eluat auf pH 7,4 äquilibriert wird, verdünnt wird und auf den Träger, insbesondere in den Röhren, inkubiert wird, wo es durch Adsorption an dem die Wand der Röhre bildenden Polymerisat fixiert wird.

12. Präsentations- oder Halterungsvorrichtung für die Röhren nach Anspruch 8, dadurch gekennzeichnet, daß sie aus thermogeformtem Plastikmaterial besteht und eine Basis (1) mit kreisförmigen, aneinanderliegenden Ausnehmungen umfaßt, die zum Einführen der Röhren einen dünnen Kanel von oben nach unten besitzen, und einen Deckel (2), der mit Hilfe zweier Einrastleisten (3) in der Basis zum Einrasten kommt, welche Einrastleisten ermöglichen, daß der Deckel zum Trocknen der Röhren oder Einführen eines Schutzgases in gehobener Position oder zum Überdecken der Röhren und zum Ermöglichen des Umdrehens derselben in gesenkter Position gehalten wird.

**Claims**

1. Process for immuno assay of antigens, employing two antibodies, consisting in fixing a second antibody on a solid phase, in incubating the latter with a solution of thu first antibody and the solution containing the antigen to be assayed to which the marked antigen is then added, characterized in that a second antibody, rendered insoluble and which the previously been purified by affinity chromatography, is used.

2. Process according to claim 1 characterized in that the reagents are added in one step without intermediate incubation, the first antibody being added last and serving as starter reagent.

3. Process according to any one of claims 1 or 2, characterized in that the human follicle-stimulating hormone and the human luteinizing hormone are assayed.

4. Process according to any one of claims 1 to 3, characterized in that the assay is carried out both at equilibrium and in kinetics.

5. Plastic supports coated with a second antibody, for carrying out the process according to claim 1, characterized in that the coating is constituted by a second antibody highly purified by affinity chromatography, fixed by adsorption on a polymer, preferably a polyethylene.

6. Supports according to claim 5, characterized in that the second antibody is a gamma-globulin of a serum of one animal species, an anti-gamma-globulin of the serum of another animal species or of man, purified by chromatography on the gamma-globulins of the second animal species or of man.

7. Supports according to any one of claims 5 and 6, characterized in that the second antibody is a sheep gamma-globulin and a rabbit anti-gamma-globulin.

8. Supports according to any one of claims 5 to 7, characterized in that they are in the form of tubes.

9. Process for preparing supports according to any one of claims 5 to 8, characterized in that the immune serum containing the second antibody is treated by affinity chromatography on gamma-globulins against which is directed the antiboby to be purified and in that the second purified antibody thus obtained is fixed by adsorption on the support, made of plastics material such as polyethylene or polystyrene.

10. Process according to claim 9, characterized in that for the affinity chromatography, a column filled with gel is used on which are fixed gamma-globulins against which the antibody to be purified is directed, and the immune serum containing the antibody to be purified is passed onto said column, then elution is carried out by a phosphate buffer at pH 7.4 up to the return of the base line of the chromatogram; the gamma-globulins of the second antibody are then eluted by a buffer bringing about a sudden variation in pH and in the conductivity of the column.

11. Process according to any one of claims 9 and 10, characterized in that the eluate containing the gamma-globulins of the second antibody is balanced at pH 7.4, diluted and incubated on the support and in particular in the tubes where it is fixed by adsorption on the polymer constituting the wall of the tube.

12. Device for presenting or holding the tubes according to claim 8, characterized in that it is constituted by a heat-formed plastics material and comprises a base (1) presenting contiguous circular cavities, having a fine downward channel for introduction of the tubes and a lid clipping in the base by means of two clipping elements which enable the lid to be maintained in top position for drying of the tubes or the introduction of a protecting gas or in low position to cover the tubes and allow them to be turned over.

Fig. 1

Fig. 2

Fig. 3